# EUROPEAN PATENT APPLICATION

(11) **EP 1 320 061 A2**
(43) Date of publication of application: **18.06.2003**
(21) Application number: 02258263.9
(22) Date of filing: 29.11.2002
(51) Int. Cl.: G06F 19/00

(54) **Fusion of computerized medical data**

(30) Priority: 13.12.2001 US 683322
(71) Applicant: GE Medical Systems Information Technologies, Inc., Milwaukee, Wisconsin 53223-3293 (US)
(72) Inventor: Reddy, Shankara B., Cedarburg, Wisconsin 53012 (US); Xue, Joel Q., Germantown, Wisconsin 53022 (US); Taha, Basel Hasan, Menomonee Falls, Wisconsin 53051 (US)
(74) Representative: Pedder, James Cuthbert

(57) **Abstract**

A method and system of diagnosing cardiac syndromes. The method includes acquiring data from a first and second diagnostic test, and then processing the data from the first and second diagnostic test to produce an first and second indicator. Next the indicators are combined and a risk of a cardiac syndrome based on the combination of indicators is calculated. The system includes a first and a second physiological activity acquisition module and a fusion engine to receive the data acquired by the modules and to generate a risk of ACS based on a combination of the data.

## Description

The present invention relates to combining various medical data to diagnose medical conditions. More specifically, the invention relates to a method and apparatus for combining medical data from various diagnostic tests to improve detection of acute cardiac syndromes ("ACS").

Early and accurate detection of ACS, such as acute myocardial infarction ("MI"), non-ST-elevated Ml, and other cardiac ischemia, is critical to reducing necrosis of cardiac tissues and the resulting mortality and morbidity. However, present diagnostic practices are often slow and inaccurate. As should be apparent, misdiagnosing or failing to promptly diagnose patients with ACS can cost lives. Yet even relatively short delays in diagnosis can lead to increased, yet non-fatal, heart damage. Further, with current methodologies and devices, patients exhibiting borderline symptoms are either unnecessarily treated for conditions they do not have or inappropriately discharged before receiving proper treatment. Both of these situations are unacceptable and lead to increased economic and human costs.

In an ideal situation, patients exhibiting signs of ACS are assessed rapidly. For example, current practice guidelines devised by the American College of Cardiology and the American Heart Association recommend that the initial assessment should be accomplished within the first ten minutes of the patient's arrival to the emergency department. The guidelines stress that no more than twenty minutes should elapse without an assessment being made. A fast diagnosis needs to take place due to the speed at which heart damage can occur.

According to the World Health Organization ("WHO"), the diagnosis of Ml should be based on the presence of at least one of three indicators; clinical history of ischemic-type chest discomfort, changes on serial electrocardiograms ("ECGs"), or the rise and fall in serum cardiac markers (biomarkers). Currently, ECG tests and the patient's history are the most commonly used diagnostic tools for screening. patients for myocardial infarction and ischemia. An elevated ST-segment present in a patient's ECG is a critical indicator of Ml, and roughly 70%-80% of patients diagnosed with Ml experience ischemic-type chest pains.

However, there is a group of patients that experience inconclusive results from these indicators. Less than 25% of patients admitted to a hospital with ischemic-type chest discomfort are diagnosed as having had an acute Ml, and roughly half of the patients diagnosed with Ml do not exhibit ST-segment elevation. There is yet another collection of patients experiencing inaccurate ECG interpretation due to human error. This error can result in up to 12% of patients being inappropriately diagnosed. When ECG and patient history tools generate such inconclusive results, physicians may turn to biomarkers to aid their diagnosis.

When a part of the heart muscle has been damaged, as in the case of Ml, certain biomarkers appear in the patient's blood at certain times. Significant levels of biomarkers are indicators of heart damage. However, the current standard for confirmation of Ml through biomarkers, testing for creatine kinase-MB (CK-MB), has several drawbacks. CK-MB testing is not likely to detect Ml in the first six to eight hours of cardiac ischemia and CK-MB remains elevated in the blood for only 72 hours. Thus, CK-MB testing is often too slow to be of use for initial assessments and the presence of CK-MB may be missed if the test is not conducted within the first few days of a cardiac event. Another biomarker, myoglobin, can be detected in the blood as early as two hours after infarction. However, the presence of myoglobin can be caused by multiple conditions (i.e., myoglobin lacks cardiac specificity). Therefore, it is recommended that a diagnosis of acute Ml be confirmed using another biomarker test, such as CK-MB or a cardiac-specific troponin. Yet, because results from biomarker tests are not available until hours after a cardiac event, the rapid assessment recommended by current guidelines can not be achieved, particularly for patients exhibiting ambiguous or borderline conditions.

Accordingly, there is a need to provide a method and an apparatus for promptly and accurately diagnosing ACS. The invention provides a method and apparatus where results from various diagnostic tests are combined or fused to improve detection of ACS. In one embodiment, the invention provides an apparatus that combines ECG data, the presence of biomarkers, and a patient's symptoms and/or history. This embodiment employs diagnostic algorithms such as combinational decision-tree logic, fuzzy logic, scoring systems, or a combination thereof. In an optional embodiment, the apparatus may be configured to accept the results of imaging tests (e.g., tests that detect cardiac perfusion) and functional assessment data as inputs. The system outputs a level of certainty of ACS (definite, probable, and possible).

In another embodiment, the invention provides a single device with physically connected inputs from an ECG acquisition module and biochemical testing module for biomarkers. The single device also outputs a level of certainty of ACS and the location of the affected myocardium.

In another embodiment of this invention, the combination of various devices (ECG acquisition module, biochemical testing module, imaging system, etc.) are electronically linked by wires, a network, or a wireless connection such as radio or infrared transmissions. This allows each device to communicate with the others and allows the devices to collectively work as if one unit.

The invention also provides a method for diagnosing ACS by acquiring data from a first and second diagnostic test, generating a first and second indicator, combining the indicators, and then calculating a risk of ACS based on that combination of indicators.

In another embodiment, the invention provides a system for diagnosing acute cardiac syndrome. The system includes a first and second device (such as an ECG acquisition module, biochemical testing module, imaging system, etc.), each generating cardiac activity data. One or more processors condition the data to produce a first and second indicator. A fusion engine receives the indicators, and generates a set of degrees of membership in overlapping classification functions based on the indicators. The engine combines the degrees of membership, and using a set of predetermined rules, generates a risk of acute cardiac syndrome.

As is apparent from the above, it is an advantage of the invention to provide a method and system for the combined interpretation of medical data and results from various diagnostic tests to improve detection of ACS. Other features and advantages of the invention will become apparent by consideration of the detailed description and accompanying drawings.

The invention will now be described in greater detail, by way of example, with reference to the drawings, in which:-
FIG. 1 is a schematic diagram of an exemplary ACS diagnostic system embodying the invention.
FIG. 2 is a graphical view of exemplary input membership functions for ECG indicators.
FIG. 3 is a graphical view of an exemplary low-risk degree of membership for ECG indicator.
FIG. 4 is a graphical view of an exemplary medium-risk degree of membership for ECG indicator.
FIG. 5 is a graphical view of an exemplary high-risk degree of membership for ECG indicator.
FIG. 6 is a graphical view of exemplary output membership functions.
FIG. 7 is a graphical representation of fuzzy logic rules.
FIG. 8 is a flowchart of an evaluation process of an exemplary ACS diagnostic system embodying the invention.

Before any embodiments of the invention are explained in detail, it is to be understood that the invention is not limited in its application to the details of construction and the arrangement of components set forth in the following description or illustrated in the following drawings. The invention is capable of other embodiments and of being practiced or of being carried out in various ways. Also, it is to be understood that the phraseology and terminology used herein is for the purpose of description and should not be regarded as limiting.

The use of "including," "comprising," or "having" and variations thereof herein is meant to encompass the items listed thereafter and equivalents thereof as well as additional items.

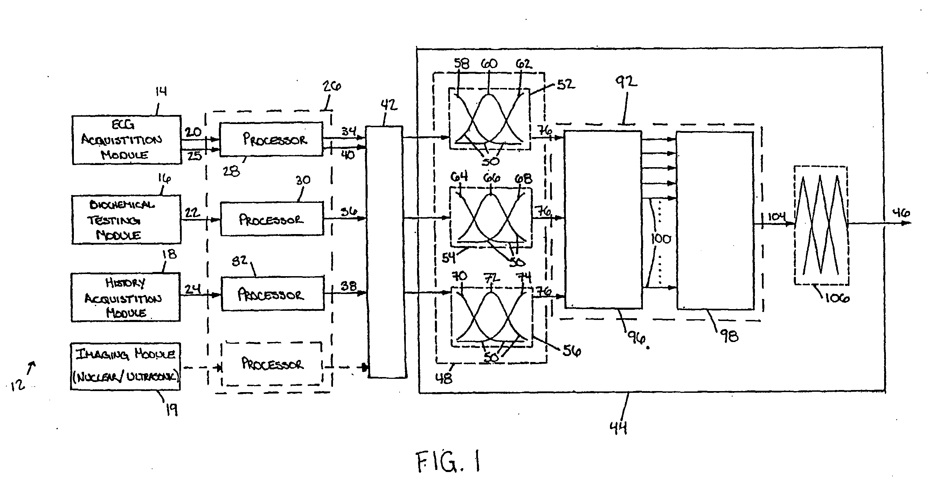

A system 10 embodying one form of the invention is illustrated in FIG. 1. The system 10 includes a plurality of cardiac activity acquisition modules or devices 12 that acquire data generated as a result of performing diagnostic tests. The system 10 employs an ECG acquisition module 14, a biochemical testing module 16, and a patient history acquisition module (or, more broadly, a history acquisition module) 18. In other embodiments, the system 10 may include any number of devices, including an imaging module 19. The imaging module 19 can be a nuclear imaging module or an ultrasonic imaging module. Also, the cardiac activity acquisition devices 12 can be generically referred to as physiological activity acquisition modules.

The ECG acquisition module 14 produces ECG data 20, such as an electrocardiogram (not shown). The biochemical testing module 16 produces biomarker data 22, and the history acquisition module 18 produces history data 24, such as patient history or trend data. The system 10 also could acquire additional data from an existing acquisition device 14, 16, or 18. In the embodiment shown, the ECG acquisition module 14 acquires additional ECG data 25, such as a second electrocardiogram. The system 10 is also capable of employing a larger number of devices as well as a broader variety of modules used to measure or monitor cardiac activity. Each of the cardiac activity acquisition devices 12 performs some sort of diagnostic test or physiological test.

Each cardiac activity acquisition device 14, 16, and 18 is coupled to a processing unit 26. The processing unit 26 conditions the ECG data 20, biomarker data 22, history data 24, and any other cardiac activity data acquired by the system 10. The processing unit 26 can take the form of a single processor or a plurality of processors. In the embodiment shown, the system 10 utilizes a plurality of processors 28, 30, and 32. Processor 28 is coupled to the ECG acquisition module 14, and generates an ECG indicator 34 from the ECG data 20. The processors 30 and 32 are coupled to the biochemical testing module 16 and the history module 18, respectively, and generates a biomarker indicator 36 and history indicator 38 from the biomarker data 22 and the history data 24. If the system 10 includes additional cardiac activity acquisition devices, such as a nuclear imaging module or an ultrasonic imaging module, then the processing unit 26 would generate corresponding indicators (not shown) from the data acquired by those devices. If an acquisition device 14, 16, or 18 produces additional data, as in the case of the second ECG data 25, the processing unit 26 generates a second ECG indicator 40 (as shown in the embodiment of FIG. 1) or combines all the ECG data 20 and 25 to produce a combined ECG indicator (not shown).

A link 42 transmits the indicators 34, 36, 38, and 40 to a fusion classification engine (or, more broadly, fusion engine) 44 from the processing unit 26. The link 42 can take the form of a wireless connection, one or more wires or similar conductors, a network, or another method of transferring data. In the embodiment shown, the fusion engine 44 fuzzifies, computes, combines, and defuzzifies the indicators 34, 36, 38, and 40 using fuzzy logic rules and a Mamdani inference method to produce a output risk 46 of cardiac syndrome. In other embodiments, the fusion engine 44 can include combinational logic, scoring systems, neutral networks, or a combination of these methods to combine the indicators 34, 36, 38, and 40 and produce an output risk 46 of cardiac syndrome.

In one embodiment, the fusion engine 44 includes a fuzzifier 48, which fuzzifies the indicators 34, 36, 38, and 40. The fuzzifier 48 includes a plurality of input classification functions (or, more broadly, input membership functions) 50, which are divided into multiple sets of functions. In the embodiment shown, the fuzzifier 48 includes three sets 52, 54, and 56 of input membership functions.

The sets 52, 54, and 56 can include any number of input membership functions 50. Each set 52, 54, and 56 can also have a different number of functions 50 compared to the other sets. In the embodiment shown, each set 52, 54, and 56 includes three input membership functions. Set 52 includes a low-risk input membership function 58, a medium-risk input membership function 60, and a high-risk input membership function 62. Set 54 includes a low-risk input membership function 64, a medium-risk input membership function 66, and a high-risk input membership function 68. Set 56 includes a low-risk input membership function 70, a medium-risk input membership function 72, and a high-risk input membership function 74. Each input membership function 58, 60, 62, 64, 66, 68, 70, 72, and 74 may take the form of a Gaussian curve and is designed specifically for a certain type of indicator.

In the embodiment shown, the fuzzifier 48 applies the first set 52 of input membership functions to the ECG indicators 34 and 40. The fuzzifier 48 also applies the second set 54 to the biomarker indicator 36, as well as the third set 56 to the history indicator 38. Instead of applying both ECG indicators 34 and 40 to one set 52 of input membership functions, in other embodiments, the fuzzifier 48 can be configured to apply a separate set (not show) of input functions to the second ECG indicator 40, while applying the first set 52 to the first ECG indicator 34.

The application of the sets 52, 54, and 56 of input membership functions to the indicators 34, 36, 38, and 40, as described above, produces a plurality of degrees of membership 76. Since the degrees of membership are produced in similar fashion, even though each input membership function results in a generally unique degree of membership, not all degrees of membership are shown in the figures. Nor are all of the degrees of membership discussed.

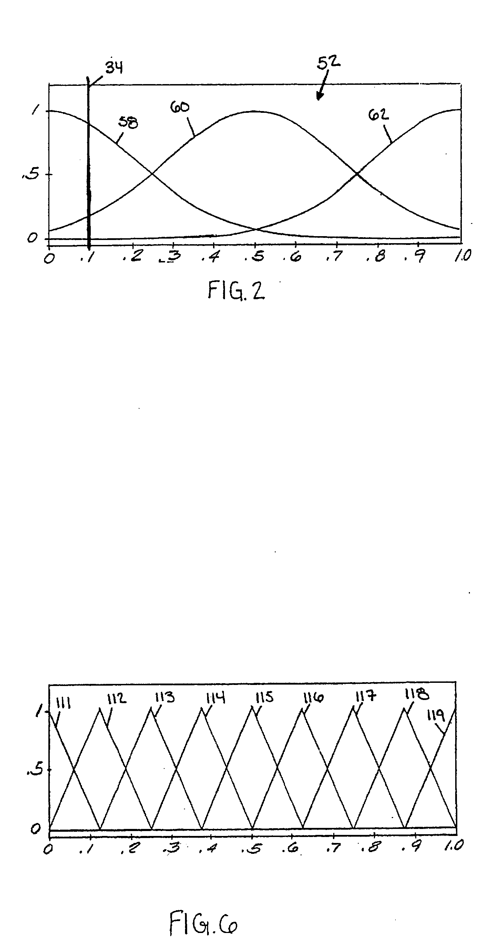

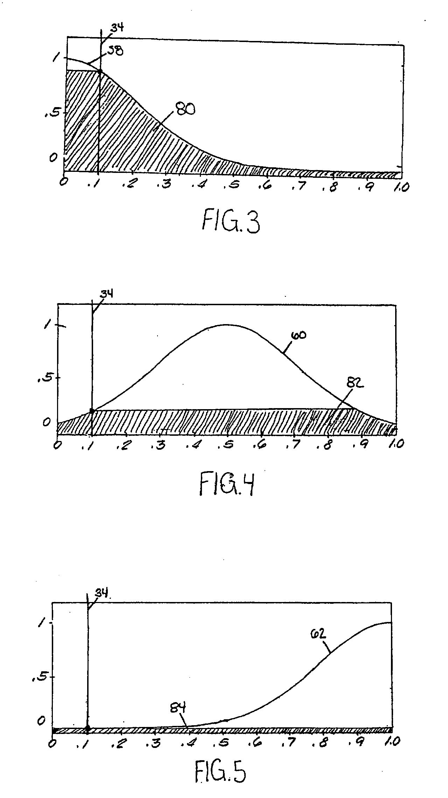

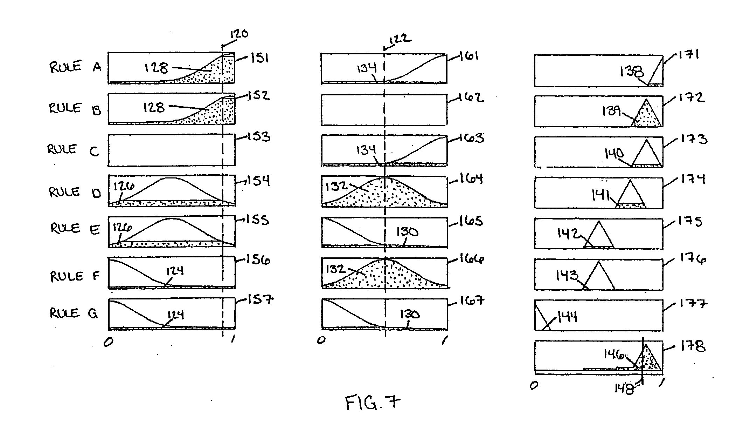

As shown in FIG. 2, the fuzzifier 48 applies the first set 52 of input membership functions to the ECG indicator 34, which is given the value of 0.1. Each input membership function 58, 60, and 62 produces a degree of membership 80, 82, and 84 from the ECG indicator 34. The low-risk input membership function 58 produces a low-risk degree of membership 80, as shown in FIG. 3. The medium-risk input membership function 60 produces a medium-risk degree of membership 82, as shown in FIG. 4; and the high-risk input membership function 62 produces a high-risk degree of membership 84, as shown in FIG. 5. Since each of the input membership functions 58, 60, and 62 overlap with the others (as shown in FIG. 2), the functions 58, 60, and 62 produce the non-zero degrees of membership 80, 82, and 84, as seen in FIGS. 3-5. In other embodiments where the input membership functions in a particular set do not overlap with the other functions, a degree of membership is still produced for each input membership function. However, that degree of membership could be zero.

Referring again to the embodiment shown in FIG. 1, the fusion engine 44 further includes an inference engine 92 coupled to the fuzzifier 48. The inference engine 92 computes and combines the degrees of membership 76 based on a plurality of diagnostic rules (shown in FIG. 7). The inference engine 92 includes a diagnostic rules applicator 96 and a diagnostic rule output combiner 98. The diagnostic rules applicator 96 evaluates each of the diagnostic rules according to all the degrees of membership 76 determined by the fuzzifier 48. The applicator 96 generates a plurality of rule outputs 100, one from each rule. After the plurality of rule outputs 100 are determined, the diagnostic rules output combiner 98 combines the plurality of rule outputs 100 to produce a combined output 104.

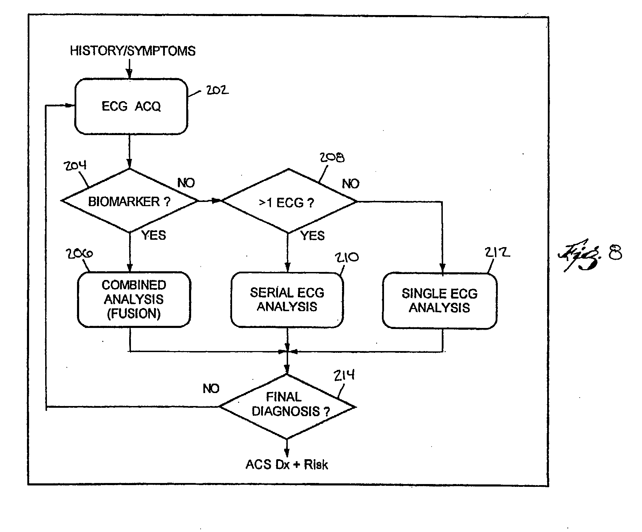

The fusion engine 44 further includes a defuzzifier 106 coupled to the diagnostic rules output combiner 98. The defuzzifier 106 includes a plurality of output membership functions 110. In the embodiment shown in FIG. 6, the defuzzifier 106 includes nine output membership functions 111, 112, 113, 114, 115, 116, 117, 118, and 119 that describe the range of varying risk of ACS. Output membership function 111 represents the least amount of risk of ACS, whereas output membership function 119 represents the most amount of risk. The remaining output membership functions 112-118 fall between functions 111 and 119, ranging from low risk to high risk, respectively. In other embodiments, the defuzzifier 106 can include more or fewer output membership functions than the embodiment shown in FIG. 6.

The defuzzifier 106 assigns the combined output 104 with an output function value based on the plurality of output membership functions 110. The output function value is calculated from the centroid method. The resulting output function value corresponds to the diagnostic risk output 46 that is produced by the system 10.

To further illustrate the stages of fuzzy logic, a graphical view of the rules at work is shown in FIG. 7. In this embodiment of the invention, the system 10 uses seven diagnostic rules A-G, reproduced in Table 1, but can employ more or fewer rules. For purposes of illustration, the fusion engine 44 is evaluating an ECG indicator 120, given a value of 0.909, and the biomarker indicator 122 (referred to in the rules as BioMarker), given a value of 0.50.

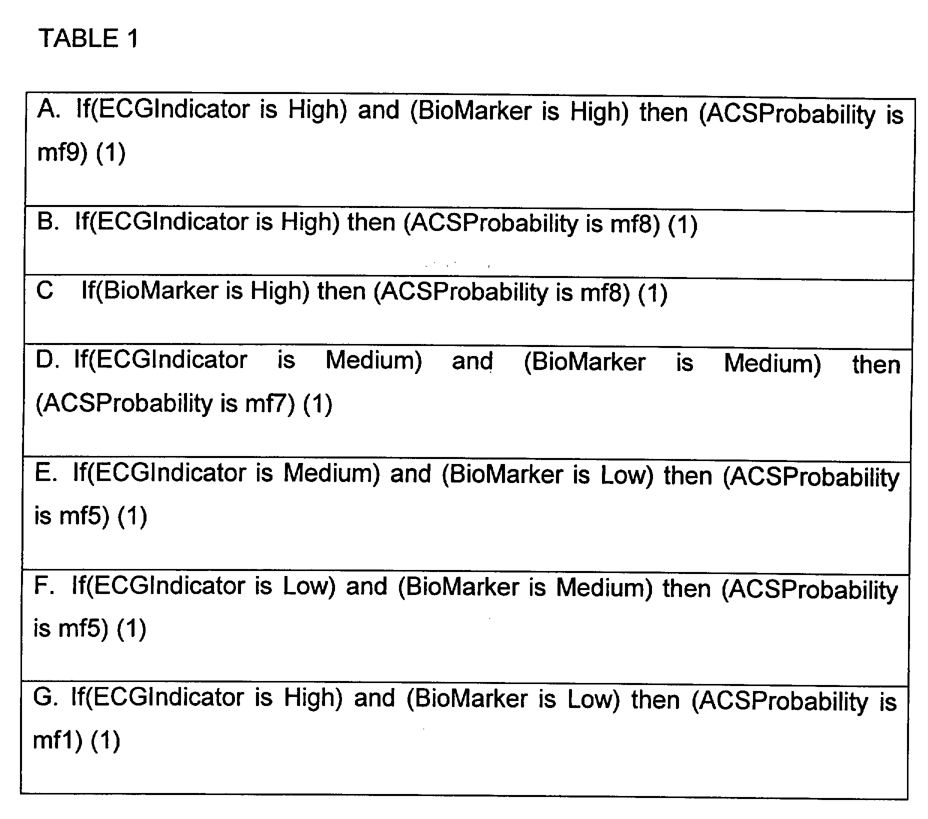

Referring to FIG. 7, the fuzzifier 48 (FIG. 1) produces a low-risk degree of membership 124, a medium-risk degree of membership 126, and a high-risk degree of membership 128 from the ECG indicator 120. The fuzzifier 48 (FIG. 1) also produces a low-risk degree of membership 130, a medium-risk degree of membership 132, and a high-risk degree of membership 134 from the biomarker indicator 122. The diagnostic rules applicator 96 (FIG. 1) collects the degrees of memberships 124, 126, 128, 130, 132, and 134 and generates a plurality of rule outputs 138-144 for rules A-G, respectively. Rule A produces output 138. Rule B produces output 139. Rule C produces output 140. Rule D produces output 141. Rule E produces output 142. Rule F produces output 143 and rule G produces output 144. The diagnostic rules output combiner 98 (FIG. 1) receives the plurality of rule outputs 138-144 from the diagnostic rules applicator 96 (FIG. 1), and constructs a combined output 146. The defuzzifier 106 (FIG. 1) proceeds to defuzzify the combined output 146 by using the centroid method or center of mass method. The centroid method and center of mass method are well-known to those of ordinary skill in the art. The result from the centroid method is the diagnostic risk output 148.

Boxes 151-157 illustrate the corresponding ECG degrees of membership 124, 126, or 128 for rules A-G, respectively. Boxes 161-167 illustrate the corresponding biomarker degrees of membership 130, 132, or 134 for rules A-G, respectively, and boxes 171-177 illustrate the corresponding rule outputs 138-144 for rules A-G, respectively. Box 178 illustrates the combined output 146 and the diagnostic risk output 148, which is calculated from the combined output 146.

For example, referring to rule B (boxes 152, 162, and 172), the output 138 is based only on the high-risk degree of membership 128 from the ECG indicator 120, as shown in box 152. Therefore, box 162 shows no biomarker degree of membership. Rule C is similar, but instead of relying solely on a degree of membership from the ECG indicator 120, rule C is based on the high-risk degree of membership 134 from the biomarker indicator 122.

As mentioned earlier, the system 10 can work with multiple cardiac activity acquisition devices 12 or in some instances a selected few. The flowchart illustrated in FIG. 8 shows the evaluating process by a system of the invention that employs a history acquisition module, an ECG acquisition module, and a biochemical testing module. After the system 10 registers the history of the patient and the symptoms being experienced, an ECG is acquired and inputted into the system as shown at step 202. At step 204, the system determines whether or not biomarker data is present. If biomarker data does exist for the patient, the system performs the combined analysis using the diagnostic rules as shown at step 206. If there is no biomarker data available for analysis, then the system determines if there is more than one set of ECG data. This occurs at step 208. If there is more than one set of ECG data, then the system performs a serial ECG analysis as shown at step 210. If there is not multiple sets of ECG data, then the system performs the single ECG analysis, shown at step 212. Each analysis proceeds to step 214, where the system determines whether or not the combined analysis is the final diagnosis. If the combined analysis is the final diagnosis, then the system outputs that diagnosis. If the combined analysis is not the final diagnosis, then the system reanalyzes the ECG data, shown at step 202, and the process repeats until a final diagnosis can be made.

Thus, the invention provides, among other things, a method and system for the combined interpretation of medical data and results from various diagnostic tests to improve detection of ACS.

For the sake of good order, various aspects of the invention are set out in the following clauses:-
1. A method of diagnosing cardiac syndromes, the method comprising the acts of:
   acquiring data (20, 22, 24, or 25) from a first diagnostic test;
   processing the data (20, 22, 24, or 25) from the first diagnostic test to produce an indicator (34, 36, 38, or 40);
   acquiring data (20, 22, 24, or 25) from a second diagnostic test;
   processing the data (20, 22, 24, or 25) from the second diagnostic test to produce a second indicator (34, 36, 38, or 40);
   combining the indicators (34, 36, 38, or 40); and
   calculating a risk (46) of a cardiac syndrome based on the combination of indicators (34, 36, 38, or 40).
2. A method as set forth in clause 1, further comprising the acts of acquiring data (20, 22, 24, or 25) from a third diagnostic test and processing the data (20, 22, 24, or 25) from the third diagnostic test to produce a third indicator (34, 36, 38, or 40).
3. A method as set forth in clause 1, wherein the act of combining the indicators (34, 36, 38, or 40) includes a Mamdani inference method.
4. A method as set forth in clause 1, wherein the act of calculating a risk (46) of a cardiac syndrome includes a Mamdani inference method.
5. A method as set forth in clause 1, wherein the act of acquiring data (20, 22, 24, or 25) from a first diagnostic test includes acquiring diagnostic data of a first type.
6. A method as set forth in clause 5, wherein the act of acquiring data (20 or 25) from a first diagnostic test is performed by an ECG acquisition module (14).
7. A method as set forth in clause 5, wherein the act of acquiring data (22) from a first diagnostic test is performed by a biochemical testing module (16).
8. A method as set forth in clause 5, wherein the act of acquiring data (24) from a first diagnostic test is performed by a history acquisition module (18).
9. A method as set forth in clause 5, wherein the act of acquiring data from a first diagnostic test is performed by a nuclear imaging module (19).
10. A method as set forth in clause 5, wherein the act of acquiring data from a first diagnostic test is performed by an ultrasonic imaging module (19).
11. A method as set forth in clause 5, wherein the act of acquiring data (20, 22, 24, or 25) from a second diagnostic test includes acquiring diagnostic data of a second type that differs from the diagnostic data (20, 22, 24, or 25) acquired by the first diagnostic test.
12. A method as set forth in clause 11, wherein the act of acquiring data (25) from a second diagnostic test includes acquiring data from an ECG acquisition module (14).
13. A method as set forth in clause 11, wherein the act of acquiring data (22) from a second diagnostic test includes acquiring data from a biochemical testing module (16).
14. A method as set forth in clause 11, wherein the act of acquiring data (24) from a second diagnostic test includes acquiring data from a history acquisition module (18).
15. A method as set forth in clause 11, wherein the act of acquiring data from a second diagnostic test includes acquiring data from a nuclear imaging module (19).
16. A method as set forth in clause 11, wherein the act of acquiring data from a second diagnostic test includes acquiring data from an ultrasonic imaging module (19).
17. A method as set forth in clause 1, wherein the method is for diagnosing acute cardiac syndromes.
18. A cardiac syndrome diagnostic system (10) comprising:
   a first cardiac activity acquisition device (14, 16, 18, 19) operable to generate a first cardiac activity data (20);
   a second cardiac activity acquisition device (14, 16, 18, 19) operable to generate a second cardiac activity data (25);
   one or more processors (26) to generate a first and second indicator (34, 40) based on the first and second cardiac activity data (20, 25), respectively; and
   a fusion engine (44) operable to receive the first and second indicators (34, 40), generate a first and second set of degrees of membership (80, 82, 84) based on the first and second indicators (34, 40), and generate a risk (46) of a cardiac syndrome based on a combination of the first and second sets of degrees of membership (80, 82, 84) and a set of predetermined rules.
19. A system (10) as set forth in clause 18, wherein the fusion engine (44) includes a fuzzifier (48).
20. A system (10) as set forth in clause 18, wherein the fusion engine (44) includes an inference engine (92).
21. A system (10) as set forth in clause 18, wherein the fusion engine (44) includes a defuzzifier (106).
22. A system (10) as set forth in clause 18, wherein the system (10) diagnoses acute cardiac syndromes.
23. A diagnostic system (10) comprising:
   a first physiological activity acquisition module (14, 16, 18, 19);
   a second physiological activity acquisition module (14, 16, 18, 19); and
   a fusion engine (44) operable to receive data (20, 22, 24, or 25) from the first and second modules (14, 16, 18, 19) and to generate a risk (46) of ACS based on a combination of the data (20, 22, 24, or 25) received from the first and second modules (14, 16, 18, 19).
24. A system (10) as set forth in clause 23, wherein the combination of the data (20, 22, 24, or 25) received from the first and second modules (14, 16, 18, 19) is based on fuzzy logic algorithms.
25. A system (10) as set forth in clause 23, wherein the first physiological activity acquisition module (14, 16, 18, 19) performs a first physiological test on physiological data (20, 22, 24, or 25) of a first type.
26. A system (10) as set forth in clause 25, wherein the first physiological activity acquisition module (12) is an ECG acquisition module (14).
27. A system (10) as set forth in clause 25, wherein the first physiological activity acquisition module (14, 16, 18, 19) is a biochemical testing module (16).
28. A system (10) as set forth in clause 25, wherein the first physiological activity acquisition module (14, 16, 18, 19) is a history acquisition module (18).
29. A system (10) as set forth in clause 25, wherein the first physiological activity acquisition module (14, 16, 18, 19) is a nuclear imaging module (19).
30. A system (10) as set forth in clause 25, wherein the first physiological activity acquisition module (14, 16, 18, 19) is an ultrasonic imaging module (19).
31. A system (10) as set forth in clause 25, wherein the second physiological activity acquisition module (14, 16, 18, 19) performs a second physiological test on physiological data (20, 22, 24, or 25) of a second type that is different than the first type of physiological data (20, 22, 24, or 25).
32. A system (10) as set forth in clause 31, wherein the second physiological activity acquisition module (14, 16, 18, 19) is an ECG acquisition module (14).
33. A system (10) as set forth in clause 31, wherein the second physiological activity acquisition module (14, 16, 18, 19) is a biochemical testing module (16).
34. A system (10) as set forth in clause 31, wherein the second physiological activity acquisition module (14, 16, 18, 19) is a history acquisition module (18).
35. A system (10) as set forth in clause 31, wherein the second physiological activity acquisition module (14, 16, 18, 19) is a nuclear imaging module (19).
36. A system (10) as set forth in clause 31, wherein the second physiological activity acquisition module (14, 16, 18, 19) is an ultrasonic imaging module (19).
37. A method for diagnosing acute cardiac syndromes ("ACS"), the method comprising the acts of:
   acquiring ECG data (20);
   processing the ECG data (20) to produce an ECG indicator (34);
   acquiring biomarker data (22);
   processing the biomarker data (22) to produce a biomarker indicator (36);
   combining the indicators (34, 36); and
   calculating a risk (46) of ACS using fuzzy logic rules.
38. A method of diagnosing cardiac syndromes, the method comprising the acts of:
   acquiring data (20, 22, 24, or 25) from a plurality of diagnostic tests;
   processing the data (20, 22, 24, or 25) from the plurality of diagnostic tests to produce a plurality of indicators (34, 36, 38, or 40);
   combining the plurality of indicators (34, 36, 38, or 40); and
   calculating a risk (46) of a cardiac syndrome based on the combination of the plurality of indicators (34, 36, 38, or 40).
39. A method as set forth in clause 38, wherein the cardiac syndrome is an acute cardiac syndrome.

## Claims

1. A method of diagnosing cardiac syndromes, the method comprising the acts of:
acquiring data (20, 22, 24, or 25) from a first diagnostic test;
processing the data (20, 22, 24, or 25) from the first diagnostic test to produce an indicator (34, 36, 38, or 40);
acquiring data (20, 22, 24, or 25) from a second diagnostic test;
processing the data (20, 22, 24, or 25) from the second diagnostic test to produce a second indicator (34, 36, 38, or 40);
combining the indicators (34, 36, 38, or 40); and
calculating a risk (46) of a cardiac syndrome based on the combination of indicators (34, 36, 38, or 40).

2. A method as set forth in claim 1, further comprising the acts of acquiring data (20, 22, 24, or 25) from a third diagnostic test and processing the data (20, 22, 24, or 25) from the third diagnostic test to produce a third indicator (34, 36, 38, or 40).

3. A method as set forth in claim 1, wherein the act of combining the indicators (34, 36, 38, or 40) includes a Mamdani inference method.

4. A cardiac syndrome diagnostic system (10) comprising:
a first cardiac activity acquisition device (14, 16, 18, 19) operable to generate a first cardiac activity data (20);
a second cardiac activity acquisition device (14, 16, 18, 19) operable to generate a second cardiac activity data (25);
one or more processors (26) to generate a first and second indicator (34, 40) based on the first and second cardiac activity data (20, 25), respectively; and
a fusion engine (44) operable to receive the first and second indicators (34, 40), generate a first and second set of degrees of membership (80, 82, 84) based on the first and second indicators (34, 40), and generate a risk (46) of a cardiac syndrome based on a combination of the first and second sets of degrees of membership (80, 82, 84) and a set of predetermined rules.

5. A system (10) as set forth in claim 4, wherein the fusion engine (44) includes a fuzzifier (48).

6. A diagnostic system (10) comprising:
a first physiological activity acquisition module (14, 16, 18, 19);
a second physiological activity acquisition module (14, 16, 18, 19); and
a fusion engine (44) operable to receive data (20, 22, 24, or 25) from the first and second modules (14, 16, 18, 19) and to generate a risk (46) of ACS based on a combination of the data (20, 22, 24, or 25) received from the first and second modules (14, 16, 18, 19).

7. A system (10) as set forth in claim 6, wherein the combination of the data (20, 22, 24, or 25) received from the first and second modules (14, 16, 18, 19) is based on fuzzy logic algorithms.

8. A method for diagnosing acute cardiac syndromes ("ACS"), the method comprising the acts of:
acquiring ECG data (20);
processing the ECG data (20) to produce an ECG indicator (34);
acquiring biomarker data (22);
processing the biomarker data (22) to produce a biomarker indicator (36);
combining the indicators (34, 36); and
calculating a risk (46) of ACS using fuzzy logic rules.

9. A method of diagnosing cardiac syndromes, the method comprising the acts of:
acquiring data (20, 22, 24, or 25) from a plurality of diagnostic tests;
processing the data (20, 22, 24, or 25) from the plurality of diagnostic tests to produce a plurality of indicators (34, 36, 38, or 40);
combining the plurality of indicators (34, 36, 38, or 40); and
calculating a risk (46) of a cardiac syndrome based on the combination of the plurality of indicators (34, 36, 38, or 40).

10. A method as set forth in claim 38, wherein the cardiac syndrome is an acute cardiac syndrome.
